# EUROPEAN PATENT SPECIFICATION

(11) **EP 4 349 348 B1**
(45) Date of publication and mention of the grant of the patent: **01.04.2026**
(21) Application number: 22810821.3
(22) Date of filing: 17.01.2022
(51) Int. Cl.: A61K 35/32, A61P 3/00, A61P 7/00, A61P 35/00, A61K 35/28, A61P 7/06, A61P 43/00

(54) **AGENT FOR USE IN IMPROVING CANCER CACHEXIA AND METHOD FOR IMPROVING CANCER CACHEXIA**
MITTEL ZUR VERWENDUNG BEI DER VERBESSERUNG DER KREBS-KACHEXIE UND VERFAHREN ZUR VERBESSERUNG VON KREBSKACHEXIE
AGENT POUR UTILISATION POUR AMÉLIORER LA CACHEXIE CANCÉREUSE ET MÉTHODE POUR AMÉLIORER LA CACHEXIE CANCÉREUSE

(30) Priority: 27.05.2021 JP 2021088875
(43) Date of publication of application: 10.04.2024
(73) Proprietor: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2022/001255
(87) International publication number: WO 2022/249535

(56) References cited:
- WO-A1-2022/054565
- JP-A- 2016 065 106
- JP-A- 2019 514 416
- JP-A- 2019 535 691
- JP-B1- 6 894 652
- US-A1- 2017 258 843
- US-A1- 2018 133 258
- ZHOU DABO ET AL: "Modification of Metal-Organic Framework Nanoparticles Using Dental Pulp Mesenchymal Stem Cell Membranes to Target Oral Squamous Cell Carcinoma", JOURNAL OF COLLOID AND INTERFACE SCIENCE, ACADEMIC PRESS,INC, US, vol. 601, 24 May 2021 (2021-05-24), pages 650 - 660, XP086719417, ISSN: 0021-9797, [retrieved on 20210524], DOI: 10.1016/J.JCIS.2021.05.126
- VAKHSHITEH FAEZEH ET AL: "Exosomes derived from miR-34a-overexpressing mesenchymal stem cells inhibit in vitro tumor growth: A new approach for drug delivery", LIFE SCIENCE, PERGAMON PRESS, OXFORD, GB, vol. 266, 9 December 2020 (2020-12-09), XP086463000, ISSN: 0024-3205, [retrieved on 20201209], DOI: 10.1016/J.LFS.2020.118871
- STANKO PETER ET AL: "Dental Mesenchymal Stem/Stromal Cells and Their Exosomes", vol. 2018, 1 January 2018 (2018-01-01), US, pages 1 - 8, XP093181259, ISSN: 1687-966X, Retrieved from the Internet <URL:http://downloads.hindawi.com/journals/sci/2018/8973613.xml> DOI: 10.1155/2018/8973613
- KONG FANXUAN ET AL: "Dental Pulp Stem Cell-Derived Extracellular Vesicles Mitigate Haematopoietic Damage after Radiation", STEM CELL REVIEWS AND REPORTS, vol. 17, no. 2, 17 April 2021 (2021-04-17), pages 318 - 331, XP037420911, ISSN: 2629-3269, DOI: 10.1007/S12015-020-10020-X
- KENNETH FEARON ET AL: "Understanding the mechanisms and treatment options in cancer cachexia", NATURE REVIEWS CLINICAL ONCOLOGY, vol. 10, no. 2, 4 December 2012 (2012-12-04), NY, US, pages 90 - 99, XP055359754, ISSN: 1759-4774, DOI: 10.1038/nrclinonc.2012.209

## Description

### Technical Field

The present invention relates to an agent for use in improving cancer cachexia.

### Background Art

Cancer cachexia, which is often called cancer anorexia-cachexia syndrome (CACS), is a multifactorial syndrome in the end stage of a cancer. A typical symptom of cancer cachexia is weight loss, and other symptoms include undernutrition, anemia and the like.

In a method for treating cachexia in the end stage of a cancer, it is required to improve the QOL (quality of life) and the ADL (activities of daily living) in the lifetime as much as possible by improving or relieving the symptoms even when the lifetime cannot be extended.

As a method for treating cachexia, PTL 3 discloses a method for treating cachexia of a patient including administering a therapeutically effective amount of anamorelin to a human cancer patient once daily for a therapeutically effective period. The publication ZHOU DABO et al. ("Modification of Metal-Organic Framework Nanoparticles Using Dental Pulp Mesenchymal Stem Cell Membranes to Target Oral Squamous Cell Carcinoma", Journal of Colloid and Interface Science, Academic Press, Inc., US, vol. 601 (24 May 2021) pages 650-660) discloses metal-organic framework nanoparticles coated with membranes of dental pulp mesenchymal stem cells. The particles are loaded with doxorubicin. The document also discloses the use of the particles to kill oral squamous cell carcinoma cells, with no appearance of cachexia.
The publication VAKHSHITEH FAEZEH et al ("Exosomes derived from miR-34a-overexpressing mesenchymal stem cells inhibit in vitro tumor growth: A new approach for drug delivery", Life Science, Pergamon Press, Oxford, GB, vol. 266 (09 Dec. 2020)) discloses the use of exosomes isolated from the culture supernatant of DPSCs to repress proliferation and migration of breast carcinoma cells in vitro.
The publication STANKO PETER et al. ("Detal Mesenchymal Stem/Stromal Cells and Their Exosomes", Stem Cells International, vol. 2018, (01 Jan. 2018), pages 1-8) discloses the use of exosomes isolated from DP-MSCs to suppress inflammation and edema in mice.
The publication KONG FANXUAN et al. ("Dental Pulp Stem Cell-Derived Extracellular Vesicles Mitigate Haematopoietic Damage after Radiation", Stem Cell Reviews And Reports, vol. 17, no. 2, pages 318-331) discloses that extracellular vesicles (EVs) derived from DPSCs have an effect on reducing haematopoietic damage.

The publication KENNETH FEARON et al., ("Understanding the mechanisms and treatment options in cancer cachexia", Nature Reviews Clinical Oncology, vol. 10, no. 2, pages 90-99) reviews current therapeutic options for the treatment of cancer cachexia, including a combination of management of nutrition, exercise and systemic inflammation.

PTL-4 (WO 2022/054565 A1) discloses a cytokine storm inhibitor comprising microparticles derived from a culture supernatant of a dental pulp-derived stem cell, an adipose derived stem cell, a bone marrow-derived stem cell, an umbilical cord-derived stem cell or an immortalized stem cell thereof, in which each of the microparticles contains an extracellular superoxide dismutase (SOD3) in an amount that is effective for the repair of a tissue or cell damaged by cytokine storm or more, the cytokine storm inhibitor being administered to a human or a non-human animal in which cytokine storm is occurring at a timing before the occurrence of the damage of the tissue by the cytokine storm and thereby inhibiting a tissue damage caused by the cytokine storm through the inhibition of the cytokine storm, or being administered to a human or a non-human animal in which the damage of a tissue by cytokine storm is occurring and thereby repairing the tissue damaged by the cytokine storm through the inhibition of the cytokine storm.

### Citation List

### Patent Literature

PTL 1: JP2019-535691A
PTL 2: JP2019-514416A
PTL 3: JP2018-154655A
PTL4: WO 2022/054565 A1

### Summary of Invention

### Technical Problem

A method for treating cachexia using a substance other than anamorelin has also been examined. PTL 3 also discloses that cachexia is treated using another small molecule compound such as megestrol acetate, melatonin and ostarine. Moreover, PTLs 1 and 2 disclose pharmaceutical compositions containing extracellular vesicles secreted from mesenchymal stem cells and cite cachexia as an example of the diseases to which the compositions are applied (claim 6 of PTL 1, [0210] of PTL 2 and the like).

A problem that the invention is to solve is to provide a novel agent for use in improving cancer cachexia.

### Solution to Problem

The present inventor has found that a culture supernatant of dental pulp-derived stem cells or microparticles such as exosomes derived from a culture supernatant can improve symptoms of cachexia of cancer cachexia model mice.

In this regard, influence of dental pulp-derived stem cells on cachexia is not disclosed in PTLs 1 and 2. In the Examples of PTL 1, it is merely described in [0227] that DP-MSC (exosomes of dental pulp-derived stem cells) behave similar to UC-(umbilical cord-derived stem cells) and CH-MSC (exosomes of chorionic placenta-derived stem cells), although with a reduced effect, and PTL 1 does not include any Example in which cachexia is examined using dental pulp-derived stem cells. PTL 2 does not include any Example using exosomes obtained from a culture supernatant of dental pulp-derived stem cells.

Specifically, the invention is defined in the appended claims.

Any reference to a method of treatment of cancer cachexia in the present description is to be interpreted as referring to a product for use in a method of treatment of cancer cachexia.

### Advantageous Effects of Invention

According to the invention, a novel agent for use in improving cancer cachexia can be provided.

### Brief Description of Drawings

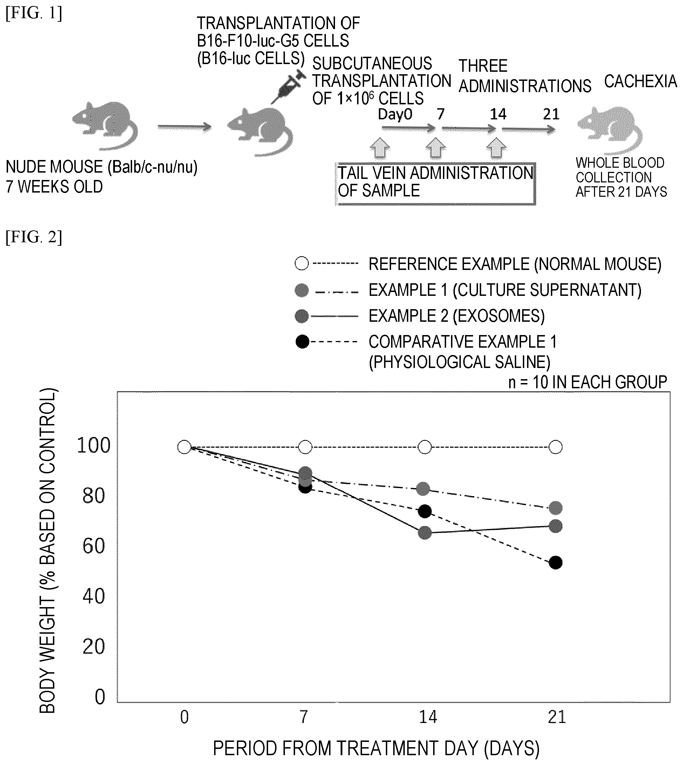

[FIG. 1] FIG. 1 shows an outline of the experiment protocol for evaluating improvement of cancer cachexia through administration of a sample to cancer cachexia model mice.
[FIG. 2] FIG. 2 is a graph showing the relations between the period (days) from the treatment day and the body weight (% based on that of the normal mice used as the control) of cancer cachexia model mice to which the agents for use in improving cancer cachexia of Examples 1 and 2 or the sample of a Comparative Example (physiological saline) was administered.

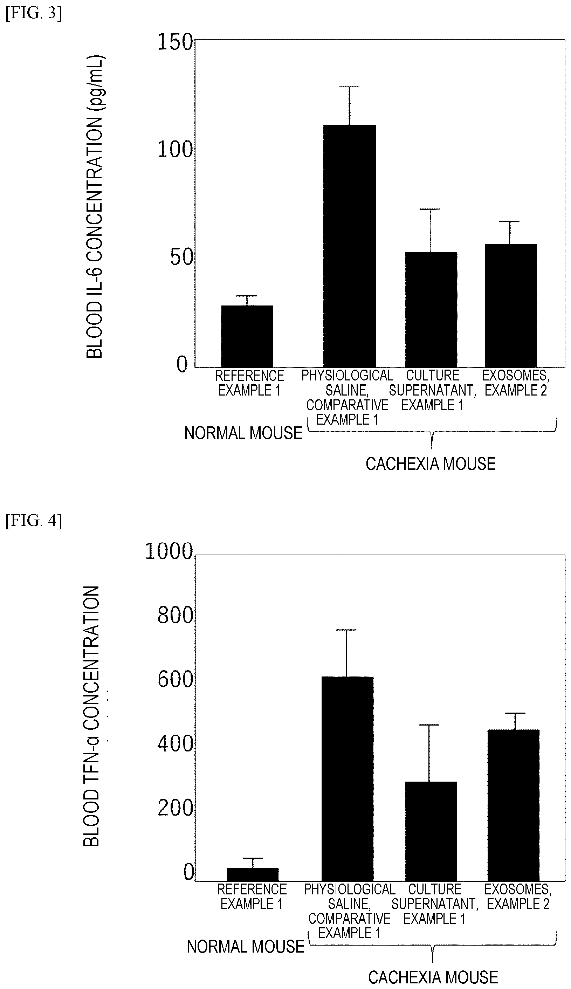

[FIG. 3] FIG. 3 is a graph showing the blood IL-6 concentrations of normal mice and cancer cachexia model mice to which the agents for use in improving cancer cachexia of Examples 1 and 2 or the sample of a Comparative Example (physiological saline) was administered.
[FIG. 4] FIG. 4 is a graph showing the blood TFN-α concentrations of normal mice and cancer cachexia model mice to which the agents for use in improving cancer cachexia of Examples 1 and 2 or the sample of a Comparative Example (physiological saline) was administered.

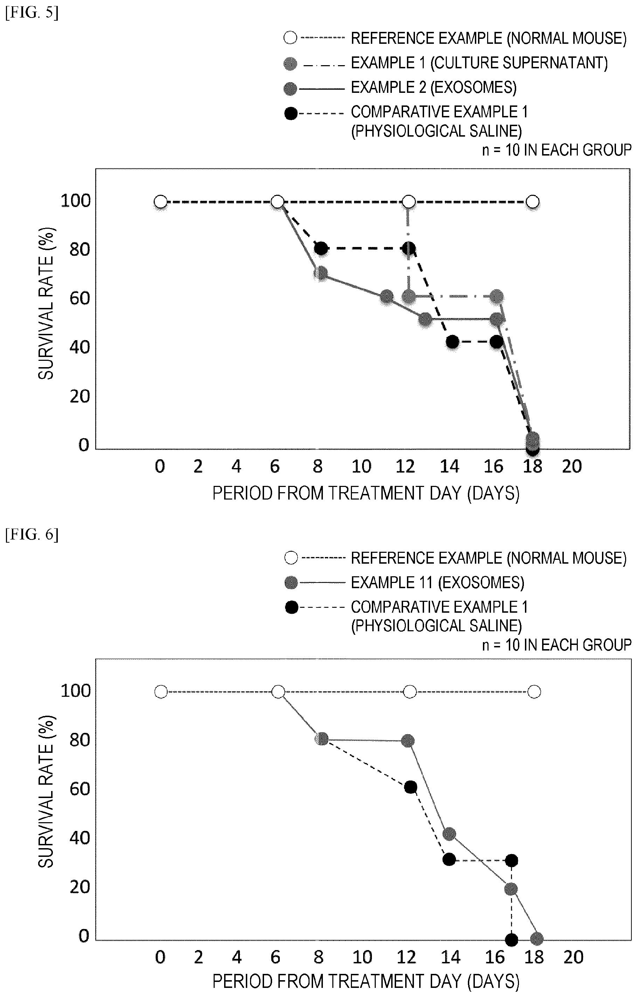

[FIG. 5] FIG. 5 is a graph showing the relations between the period (days) from the treatment day and the survival rate of normal mice and cancer cachexia model mice to which the agents for use in improving cancer cachexia of Examples 1 and 2 or the sample of a Comparative Example (physiological saline) was administered.
[FIG. 6] FIG. 6 is a graph showing the relations between the period (days) from the treatment day and the survival rate of normal mice and cancer cachexia model mice to which 1.25 times the dose of the agent for use in improving cancer cachexia of Example 2 was given or the sample of a Comparative Example (physiological saline) was administered.

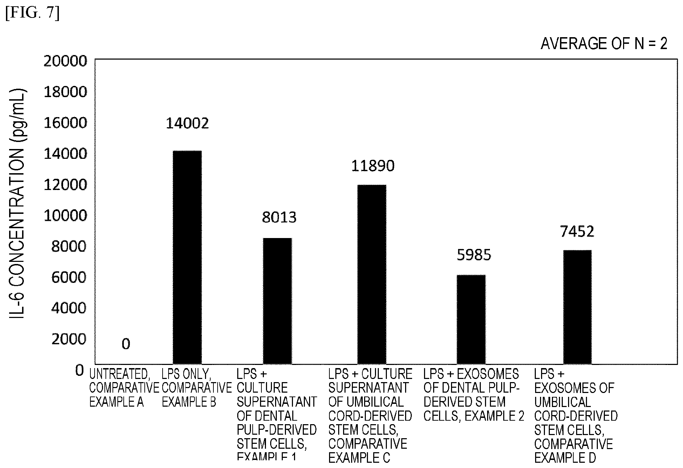

[FIG. 7] FIG. 7 is a graph showing the concentrations of IL-6 released into the culture solutions after culture of LPS stimulation-induced active macrophage cells, which are a main cause of a cytokine storm, without treatment (Comparative Example A) or with administration of the agents for use in improving cancer cachexia of Examples 1 and 2 or the samples of Comparative Examples (physiological saline of Comparative Example B or the culture supernatant or the exosomes of umbilical cord-derived stem cells of Comparative Examples C and D).

### Description of Embodiments

The invention is explained in detail below. The constituent features described below are sometimes explained based on typical embodiments or specific examples.

In the specification, a numerical range expressed using "to" means a range including the values before and after "to" as the lower limit and the upper limit.

### [Agent for Use in Improving Cancer Cachexia]

In a first aspect, the agent for use in improving cancer cachexia of the invention is an agent for use in improving cancer cachexia by administration to a subject with cancer cachexia which is a composition containing a culture supernatant of dental pulp-derived stem cells, and the culture supernatant contains microparticles derived from the dental pulp-derived stem cells.

In a second aspect, the agent for use in improving cancer cachexia of the invention is an agent for use in improving cancer cachexia by administration to a subject with cancer cachexia which is a composition containing microparticles derived from a culture supernatant of dental pulp-derived stem cells, and the composition does not contain the culture supernatant from which the microparticles have been removed.

To improve the effect of improving cancer cachexia, the agent for use in improving cancer cachexia of the invention is more preferably a composition containing a culture supernatant of dental pulp-derived stem cells, particularly preferably a culture supernatant of dental pulp-derived stem cells.

Preferable aspects of the agent for use in improving cancer cachexia of the invention are explained below.

### <Definitions of Cancer Cachexia>

Cancer cachexia means cachexia of a subject with a cancer. The agent for use in improving cancer cachexia of the invention improves cancer cachexia but may also be able to improve cachexia other than cancer cachexia.

The "cachexia" can be defined by various methods in any of the major embodiments or the more specific embodiments of the invention. In particular, any one of the following definitions can be used.
(1) A clinical syndrome characterized by one or a combination of anorexia, early satiety, weight loss, muscle fatigue, undernutrition, anemia and edema, preferably a clinical syndrome defined by three, four, five or all of the symptoms.
(2) Weight loss of 5% or more in the past six months and/or a body mass index of less than 20 kg/m².
(3) BMI<20 and weight loss exceeding 2% in the past three or six months.
(4) Weight loss>2% in the past three or six months for a limb skeletal muscle mass index equivalent to that of sarcopenia (male<7.26 kg/m²; female<5.45 kg/m²).
(5) A multifactorial syndrome characterized by severe decreases in the body weight, fat and muscles and an increase in protein catabolism caused by an underlying disease.

Regarding the weight loss, when the agent for use in improving cancer cachexia of the invention is used for treating weight loss of a subject with cancer cachexia, the agent can preferably treat a subject with weight loss of 2% or more in the past 14 days.

Early satiety refers to a tendency of feeling bloating or satiety early after taking a meal.

Fatigue is generally defined as feeling of weariness, tiredness or lack of energy. Fatigue can also be defined from the patient's score regarding various types of evaluation or self-evaluation including questions designed to rank feeling of weariness, tiredness or lack of energy. Specific evaluation includes Functional Assessment of Cancer Therapy -General (FACT-G) for 27 items and FACIT-F including Fatigue Subscale of 13 questions which are scored on a scale of 0-4 and which can assess the patient's perception for fatigue- and anemia-related concerns.

### <Improvement of Symptoms of Cancer Cachexia>

In the invention, of the symptoms of cancer cachexia, weight loss, undernutrition and anemia can be preferably improved in particular. It is required to improve the QOL and the ADL in the lifetime by improving the symptoms. In particular, when the subject with cancer cachexia has a terminal cancer, it is preferable that the subject can die peacefully while weight loss, undernutrition and anemia are suppressed, relieved or controlled until the moment of death.

Hereinafter, explanation is made together with the mechanism of action of the agent for use in improving cancer cachexia.

### <Mechanism of Action of Agent for Use in Improving Cancer Cachexia>

### (Suppression of Blood Cytokines)

By suppressing blood cytokines, weight loss, anemia and undernutrition, of the symptoms of cachexia, are improved.

Cytokine is a general name for proteins involved in inflammatory reaction and immune response, and several hundred kinds or more are known. Cytokines each have various activities, and well-balanced, coordinated interaction thereof maintains or regulates biological functions. Cytokines are generally produced/released through immune response to infection or the like, but in cancer cachexia, the blood concentrations of two or more cytokine kinds increase abnormally due to uncontrolled production/release of the cytokines from tumor cells. In particular, inflammatory cytokines are involved in pathological conditions of cancer cachexia, and IL-1, IL-6, TNFα and IFN-γ are especially important. Examples of other cytokines to be suppressed include IL-2, IL-4, IL-10, IL-17, IL-18, MIG, MIP-1α and the like. The cytokines released from tumor cells activate immune/inflammatory cells of the host through promotion of inflammatory reaction at the tumor site and cause a so-called cytokine storm. Cancer cachexia is characterized by a pathological condition of weight loss due to a decrease in skeletal muscles.

IL-1 is an inflammatory cytokine involved in inflammation and protection against infection.

IL-2 is a cytokine involved in cellular immunity. Excessive expression of IL-2 leads to a selective increase in regulatory T cells (Tregs), which are a subset of T cells. Tregs exhibit an action of maintaining peripheral tolerance by suppressing immune response of other cells. Breakdown of peripheral tolerance is believed to cause autoimmune diseases in humans. Thus, the immunosuppressive ability of Tregs is believed to prevent the development of autoimmune diseases. Moreover, Tregs are involved also in cancer, and solid tumors and hematologic malignancies are associated with elevated numbers of Tregs (see [0007] of JP2020-002154A).

IL-4 is a non-redundant cytokine involved in the differentiation of T helper cells into the Th2 (T helper type 2) subset, and Th2 promotes the differentiation of premature B cells into IgE-producing plasma cells. The IgE level is elevated in allergic asthma. Thus, IL-4 is involved in the development of allergic asthma (see [0008] of JP2020-002154A).

IL-6 is a B cell differentiation factor, a B cell-stimulating factor-2, a hepatocyte-stimulating factor, a hybridoma growth factor and a plasmacytoma growth factor. IL-6 is a typical inflammatory cytokine and is a multifunctional cytokine involved in regulation of acute inflammatory response, modulation of specific immune responses including B and T cell differentiation, bone metabolism, thrombopoiesis, epidermal proliferation, menses, neuronal cell differentiation, neuroprotection, aging, cancer, inflammatory reaction occurring in Alzheimer's disease and the like. IL-6 is believed to play a role in the development of many diseases and disorders, including fatigue, cachexia, autoimmune diseases, diseases of the skeletal system, cancer, heart disease, obesity, diabetes, asthma, Alzheimer's disease and multiple sclerosis (see [0002] to [0005] of JP2019-047787A).

IL-10 is a homodimer protein having 160 amino acid residues produced in T cells, macrophages and dendric cells. IL-10 is known to be involved in suppression of macrophage functions, B cell activation and the like (see [0052] of JP2018-212237A). Moreover, IL-10 is an anti-inflammatory cytokine.

IL-17 is a protein having 132 amino acid residues produced in CD4 memory T cells and Th17 cells. IL-17 is known to be involved in inflammatory cytokine production from macrophages, epithelial cells, endothelial cells and fibroblasts and the like (see [0053] of JP2018-212237T).

IL-18 is released when a severe stress is applied to cells due to viral infection, cancer reactive oxygen species or the like.

IFNs (interferons) have the functions of inhibiting viral replication and activating immune cells (for example, natural killer cells, macrophages and the like) and the like. IFNs are divided into type I IFNs, type II IFNs and type III IFNs. Of these, a type II IFN, IFNγ, is a pleiotropic cytokine which is produced by activated immune cells and can induce cellular responses such as an increase in macrophage activity, an increase in the expression of MHC molecules and an increase in NK cell activity (see [0209] of JP2020-522254A).

TNF (tumor necrosis factor) is a typical inflammatory cytokine. TNFα, TNFβ and LTβ are known as TNFs. Of these, TNFα mediates a number of important vital functions, including structural and functional organization of secondary lymphoid organs, apoptosis and antitumor activity, inhibition of viral replication, immunoregulation and inflammation. TNFs also play important roles in pathogenesis of autoimmune diseases, acute phase reaction, septic shock, fever and cachexia (see [0004] of JP2020-079306A).

MIG is a cytokine which governs type 1 immune response. MIG is produced by macrophages, epithelial cells, vascular endothelial cells and the like and causes Th1 cells, CD8T cells, some macrophages and the like to migrate to an inflammation site.

MIP-1α is a cytokine produced by macrophages, fibroblasts, epithelial cells, vascular smooth muscle cells and the like in response to inflammation.

Of these, the agent for use in improving cancer cachexia of the invention is preferably used for suppressing increases in the IL-6 concentration and the TFN-α concentration in the blood of a subject with cancer cachexia.

### (Improvement of Amounts of Hemocytes and the Like in Blood)

The agent for use in improving cancer cachexia of the invention can preferably improve the amounts of hemocytes and the like in the blood and can more preferably improve a red blood cell count, a hemoglobin level (the blood pigment count), a platelet count and a hematocrit. The agent for use in improving cancer cachexia of the invention is particularly preferably used for suppressing decreases in the red blood cell count, the hemoglobin level and the hematocrit of a subject with cancer cachexia. By improving these, undernutrition and anemia are improved.

### (Types of Cancers)

The agent for use in improving cancer cachexia of the invention can be applied to any type of cancer, but the agents are each preferably generally applied to a cancer of type with cancer cachexia. Non-limiting examples of related cancers include, for example, breast cancer, prostate cancer, multiple myeloma, transitional cell cancer, lung cancer (for example, non-small-cell lung cancer (NSCLC)), renal cancer, thyroid cancer, other cancers causing hyperparathyroidism, adenocarcinoma, leukemia (for example, chronic myeloid leukemia, acute myeloid leukemia, chronic lymphocytic leukemia and acute lymphocytic leukemia), lymphoma (for example, B-cell lymphoma, T-cell lymphoma, non-Hodgkin's lymphoma and Hodgkin's lymphoma), head and neck cancer, esophageal cancer, stomach cancer, colon cancer, bowel cancer, colorectal cancer, rectal cancer, pancreatic cancer, liver cancer, cholangiocarcinoma, gallbladder cancer, ovarian cancer, endometrial cancer, vaginal cancer, cervical cancer, bladder cancer, neuroblastoma, sarcoma, osteosarcoma, malignant melanoma, squamous cell carcinoma, bone cancers including both primary bone cancer (for example, osteosarcoma, chondrosarcoma, Ewing sarcoma, fibrosarcoma, malignant fibrous histiocytoma, adamantinoma, giant cell tumor and chordoma) and secondary (metastatic) bone cancer, soft tissue sarcoma, basal cell carcinoma, angiosarcoma, hemangiosarcoma, myxosarcoma, liposarcoma, osteosarcoma (osteogenic sarcoma), angiosarcoma, endothelial sarcoma, lymphangiosarcoma, lymphatic endothelial sarcoma, synovioma, testicular cancer, uterine cancer, gastrointestinal cancer, mesothelioma, leiomyosarcoma, rhabdomyosarcoma, adenocarcinoma, sweat gland cancer, sebaceous adenocarcinoma, papillary cancer, Waldenstroma macroglobulinemia, papillary adenocarcinoma, cystadenocarcinoma, bronchial carcinoma, choriocarcinoma, seminoma, embryonal carcinoma, Wilms tumor, epithelial cancer, glioma, glioblastoma, astrocytoma, medulloblastoma, craniopharyngioma, ependymoma, pinealoma, hemangioblastoma, acoustic neuroma, oligodendroglioma, meningioma, retinoblastoma, medullary carcinoma, thymoma and the like. In a preferable embodiment, the cancer is non-small-cell lung cancer (NSCLC), and the cancer is further preferably unresectable NSCLC in phase III or phase IV.

### <Components of Agent for Use in Improving Cancer Cachexia>

The active ingredient of the agent for use in improving cancer cachexia of the invention is a culture supernatant of dental pulp-derived stem cells or microparticles derived from a culture supernatant of dental pulp-derived stem cells.

Compared to the conventional compositions which can be used as agents for improving cancer cachexia, the agent for use in improving cancer cachexia of the invention has advantages such as easiness of mass production, utilization of culture solutions of stem cells which are otherwise disposed of as industrial waste or the like and decreased cost of the disposal of culture solutions of stem cells. In particular, when the culture supernatant of dental pulp-derived stem cells is a culture supernatant of human dental pulp-derived stem cells, the agent for use in improving cancer cachexia of the invention also has advantages of high safety for example from the immunological point of view and less ethical issues in application to a human. When the culture supernatant of dental pulp-derived stem cells is a culture supernatant of dental pulp-derived stem cells of a patient with cancer cachexia, the application of the agent for use in improving cancer cachexia of the invention to such a patient will be safer and cause less ethical issues.

The agent for use in improving cancer cachexia of the invention is derived from a culture supernatant of dental pulp-derived stem cells and thus is used also for applications in restorative medicine. In particular, a composition containing microparticles derived from a culture supernatant of dental pulp-derived stem cells or the like is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by a culture supernatant of dental pulp-derived stem cells or a composition using microparticles derived therefrom. Compared to stem cell transplantation, the case using the agent for use in improving cancer cachexia of the invention does not easily cause neoplastic transformation or the like and is considered to be safer because cell transplantation is not required. Moreover, the agent for use in improving cancer cachexia of the invention has advantages because one with uniformly standardized quality can be used. Because mass production and an efficient administration method can be selected, use with a low cost is enabled.

### (Culture Supernatant of Dental Pulp-Derived Stem Cells)

### - Dental Pulp-Derived Stem Cells -

The culture supernatant of dental pulp-derived stem cells or the like is not particularly restricted.

The culture supernatant of dental pulp-derived stem cells or the like preferably does not substantially contain serum. For example, the serum content of the culture supernatant of dental pulp-derived stem cells or the like is preferably 1 mass% or less, more preferably 0.1 mass% or less, particularly preferably 0.01 mass% or less.

The dental pulp-derived stem cells may be derived from a human or from a nonhuman animal. The nonhuman animal may be the same as the animal (species) as the subject of the administration of the agent for use in improving cancer cachexia of the invention described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used. In addition to human deciduous dental pulp stem cells and human permanent dental pulp stem cells, dental pulp-derived stem cells derived from a nonhuman animal such as pig deciduous dental pulp stem cells can be used.

The dental pulp-derived stem cells can produce, in addition to exosomes, vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-α, KGF, HBEGF, SPARC, other growth factors and various cytokines such as chemokine. Many other bioactive substances can also be produced.

In the invention, the dental pulp-derived stem cells used for the culture supernatant of dental pulp-derived stem cells are particularly preferably dental pulp-derived stem cells containing many proteins, and deciduous dental pulp stem cells are preferably used. That is, in the invention, a culture supernatant of deciduous dental pulp stem cells is preferably used.

The dental pulp-derived stem cells used in the invention may be natural cells or genetically modified cells as long as the intended treatment can be achieved.

In particular, in the invention, immortalized stem cells of dental pulp-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells are not particularly restricted. The immortalized stem cells are preferably non-tumor immortalized stem cells. The immortalized stem cells of dental pulp-derived stem cells can be prepared by adding one of the following small molecule compounds (inhibitors) or any combination thereof to dental pulp-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole and 2-(3-(6-methylpyridin-2-yl)-1H-pyrazol-4-yl)-1,5-naphthyridine (all are from Merck), SB431542 (Sigma-Aldrich) and the like. A-83-01 is preferable.

ROCK inhibitors are not particularly limited as long as the ROCK inhibitors have the action of inhibiting the function of Rho-binding kinase. Examples of the ROCK inhibitors include GSK269962A (Axonmedchem), Fasudil hydrochloride (Tocris Bioscience), Y-27632 and H-1152 (both are from FUJIFILM Wako Pure Chemical Corporation) and the like. Y-27632 is preferable.

GSK3 inhibitors are not particularly limited as long as GSK-3 (glycogen synthase kinase 3) is inhibited, and examples thereof include A 1070722, BIO and BIO-acetoxime (all are from TOCRIS) and the like.

MEK inhibitors are not particularly limited as long as the MEK inhibitors have the action of inhibiting the function of MEK (MAP kinase-ERK kinase), and examples thereof include AZD6244, CI-1040 (PD184352), PD0325901, RDEA119 (BAY86-9766), SL327 and U0126-EtOH (all are from Selleck), PD98059, U0124 and U0125 (all are from Cosmo Bio Co., Ltd.) and the like.

When the agent for use in improving cancer cachexia of the invention is used for regenerative medicine, due to the requirements of the Act on the Safety of Regenerative Medicine, the culture supernatant of dental pulp-derived stem cells or immortalized stem cells thereof or the composition containing microparticles derived therefrom does not contain other somatic stem cells than the dental pulp-derived stem cells or the like. The agent for use in improving cancer cachexia of the invention may contain mesenchymal stem cells other than the dental pulp-derived stem cells or the like or other somatic stem cells but preferably does not contain such cells.

Examples of the somatic stem cells other than the mesenchymal stem cells include stem cells derived from the dermal system, the digestive system, the myeloid system, the nerve system and the like, but the somatic stem cells are not limited to the examples. Examples of the somatic stem cells of the dermal system include epithelial stem cells, hair follicle stem cells and the like. Examples of the somatic stem cells of the digestive system include pancreatic (general) stem cells, hepatic stem cells and the like. Examples of the somatic stem cells of the myeloid system (except for the mesenchymal stem cells) include hematopoietic stem cells and the like. Examples of the somatic stem cells of the nerve system include neural stem cells, retinal stem cells and the like.

The agent for use in improving cancer cachexia of the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

### - Preparation Method of Culture Supernatant of Dental Pulp-Derived Stem Cells -

The method for preparing the culture supernatant of dental pulp-derived stem cells or immortalized stem cells thereof is not particularly restricted, and a conventional method can be used.

The culture supernatant of dental pulp-derived stem cells or the like is a culture solution obtained by culturing dental pulp-derived stem cells. For example, a culture supernatant which can be used for the invention can be obtained by culturing dental pulp-derived stem cells and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used. In particular, the culture supernatant is preferably a culture supernatant containing microparticles obtained by subjecting a culture solution obtained by culturing dental pulp-derived stem cells to centrifugation to such an extent that the dental pulp-derived stem cells or dead cells thereof can be removed. After the centrifugation, a specific fraction containing a large amount of the microparticles may be collected to prepare the culture supernatant.

The dental pulp-derived stem cells for obtaining the culture supernatant of dental pulp-derived stem cells can be selected by a normal method and can be selected based on the size and the morphology of the cells or as adherent cells. The dental pulp-derived stem cells can be selected from dental pulp cells collected from an exfoliated deciduous tooth or a permanent tooth as adherent cells or passaged cells thereof. The culture supernatant of dental pulp-derived stem cells used can be a culture supernatant obtained by culturing selected stem cells.

The "culture supernatant of dental pulp-derived stem cells or the like" is preferably a culture solution which does not contain the cells themselves obtained by culturing the dental pulp-derived stem cells or the like. The culture supernatant of dental pulp-derived stem cells used in the invention preferably does not contain any cells (regardless of the kinds of cells) as a whole in an aspect. Due to the feature, the composition of the aspect is clearly distinguished of course from the dental pulp-derived stem cells themselves and also from various compositions containing dental pulp-derived stem cells. A typical example of the aspect is a composition which does not contain dental pulp-derived stem cells and which is composed only of a culture supernatant of dental pulp-derived stem cells.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is further particularly preferably a composition composed only of a culture supernatant of deciduous dental pulp-derived stem cells.

A basal medium, a basal medium supplemented with serum and the like or the like can be used for the culture solution of dental pulp-derived stem cells for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F12 medium (SIGMA, GIBCO and the like), RPMI1640 medium and the like. Examples of the component that can be added to the medium include serum (fetal bovine serum, human serum, sheep serum and the like), serum replacements (knockout serum replacement (KSR) and the like), bovine serum albumin (BSA), antibiotics, vitamins and minerals.

In order to prepare "a culture supernatant of dental pulp-derived stem cells" which does not contain serum, however, a serum-free medium should be used throughout the whole process or for the last passaging culture or the last passaging culture steps. For example, when dental pulp-derived stem cells are cultured in a medium which does not contain serum (a serum-free medium), a culture supernatant of dental pulp-derived stem cells which does not contain serum can be prepared. When passaging culture is conducted once or two or more times and when the last passaging culture or the last passaging culture steps are conducted in a serum-free medium, a culture supernatant of dental pulp-derived stem cells or the like which does not contain serum can also be obtained. Moreover, a culture supernatant of dental pulp-derived stem cells which does not contain serum can also be obtained by removing serum from the recovered culture supernatant using dialysis, solvent substitution using a column or the like.

For culturing the dental pulp-derived stem cells for obtaining the culture supernatant, generally used conditions can be applied as they are. The method for preparing a culture supernatant of the dental pulp-derived stem cells can be the same as the cell culture method described below except that the isolation and selection steps of the stem cells are appropriately adjusted to the type of the stem cells. One skilled in the art can appropriately isolate and select dental pulp-derived stem cells according to the type of the dental pulp-derived stem cells.

In order to produce a large amount of exosomes, special conditions may be applied for culturing the dental pulp-derived stem cells. The special conditions may be, for example, low-temperature conditions, low-oxygen conditions, microgravity conditions, conditions of coculturing with any stimulant or the like.

The culture supernatant of dental pulp-derived stem cells used for preparing exosomes in the invention may contain another component in addition to the culture supernatant of dental pulp-derived stem cells but preferably does not substantially contain any other component.

The culture supernatant of dental pulp-derived stem cells may be stored after an additive used for preparing exosomes is added to the culture supernatant.

### (Microparticles)

The microparticles are derived from dental pulp-derived stem cells, for example, through secretion, emergence, dispersion or the like from the dental pulp-derived stem cells and are exuded or released or fall into a cell culture medium. Thus, the microparticles are contained in a culture supernatant of dental pulp-derived stem cells.

The microparticles derived from a culture supernatant of dental pulp-derived stem cells may be used in the state of being contained in the culture supernatant or used in the state purified from the culture supernatant. The microparticles are preferably microparticles purified from the culture supernatant.

The origin of the microparticles can be identified by a known method. For example, the stem cell origin of the microparticles, such as dental pulp-derived stem cells, adipose-derived stem cells, bone marrow-derived stem cells and umbilical cord-derived stem cells, can be identified by the method described in J Stem Cell Res Ther (2018) 8:2. Specifically, based on the miRNA pattern of the microparticles, the origin of the microparticles can be identified.

According to the invention the microparticles are at least one kind selected from the group consisting of exosomes, membrane vesicles, ectosomes, exovesicles and microvesicles, more preferably exosomes.

The diameter of the microparticles is preferably 10 to 1000 nm, more preferably 30 to 500 nm, particularly preferably 50 to 150 nm.

Moreover, molecules called tetraspanins such as CD9, CD63 and CD81 are desirably on the surface of the microparticles, and the molecules may be those of CD9 alone, CD63 alone, CD81 alone or any combination of two or three thereof.

A preferable aspect in which exosomes are used as the microparticles is sometimes explained below, but the microparticles used for the invention are not limited to exosomes.

The exosomes are preferably extracellular vesicles which are released from cells during fusion of plasma membrane and multivesicular bodies.

The surface of the exosomes preferably contains a lipid and a protein derived from the cell membrane of dental pulp-derived stem cells.

The exosomes preferably contain, in their inside, an intracellular substance of dental pulp-derived stem cells such as nucleic acids (microRNAs, messenger RNAs, DNAs and the like) and proteins.

The exosomes are known to be used for communication among cells through transportation of genetic information from a cell to another cell. The exosomes can be tracked easily and can be targeted at a specific region.

### - Microparticle Content -

The microparticle content of the agent for use in improving cancer cachexia of the invention is not particularly restricted. The agent for use in improving cancer cachexia of the invention preferably contains 0.5×10⁸ particles or more, more preferably 1.0×10⁸ particles or more, particularly preferably 2.0×10⁸ particles or more, further particularly preferably 2.5×10⁸ particles or more, still further particularly preferably 1.0×10⁹ particles or more of the microparticles.

The concentration of the microparticles contained in the agent for use in improving cancer cachexia of the invention is not particularly restricted. The agent for use in improving cancer cachexia of the invention preferably contains the microparticles at 1.0×10⁸ particles/mL or more, more preferably at 2.0×10⁸ particles/mL or more, particularly preferably at 4.0×10⁸ particles/mL or more, further particularly preferably at 5.0×10⁸ particles/mL or more, still further particularly preferably at 2.0×10⁹ particles/mL or more.

In a preferable aspect of the agent for use in improving cancer cachexia of the invention, the amounts of hemocytes and the like in the blood can be maintained high, and the amounts of cytokines (in particular, inflammatory cytokines) can be suppressed, resulting in a tendency towards some extension of lifetime, because the microparticles are contained in a large amount or at a high concentration in this manner.

### - Preparation Method of Microparticles -

The microparticles can be prepared by purifying the microparticles from the culture supernatant of dental pulp-derived stem cells or the like.

The purification of the microparticles is preferably separation of a fraction containing the microparticles from the culture supernatant of dental pulp-derived stem cells, more preferably isolation of the microparticles.

The microparticles can be isolated by separation from non-associated components based on the characteristics of the microparticles. For example, the microparticles can be isolated based on the molecular weight, the size, the morphology, the composition or the biological activity.

In the invention, the microparticles can be purified by centrifuging the culture supernatant of dental pulp-derived stem cells and collecting a specific fraction containing a large amount of the microparticles (for example, precipitates) obtained by the centrifugation. Unnecessary components (insoluble components) in a faction other than the predetermined fraction may be removed. The solvent, the dispersion medium and the unnecessary components do not have to be removed completely from the agent for use in improving cancer cachexia of the invention. An example of the centrifugation conditions is 100 to 20000 g for 1 to 30 minutes.

In the invention, the microparticles can be purified by subjecting the culture supernatant of dental pulp-derived stem cells or a centrifuged product thereof to filtration. Unnecessary components can be removed through the filtration. Moreover, when a filter membrane having an appropriate pore size is used, the removal of the unnecessary components and sterilization can be conducted simultaneously. The material, the pore size and the like of the filter membrane used for the filtration are not particularly limited. The filtration can be achieved by a known method using a filter membrane having an appropriate molecular weight or an appropriate size cut-off. Because exosomes can be easily fractionated, the pore size of the filter membrane is preferably 10 to 1000 nm, more preferably 30 to 500 nm, particularly preferably 50 to 150 nm.

In the invention, the culture supernatant of dental pulp-derived stem cells, a centrifuged product thereof or a filtered product thereof can be separated further using separation means such as column chromatography. For example, high-performance liquid chromatography (HPLC) using any of various columns can be used. The column which can be used is a size exclusion column or a binding column.

In order to track the microparticles (or the activity thereof) in the fraction at each treatment stage, one or more characteristics or the biological activity of the microparticles can be used. For example, in order to track the microparticles, light scattering, refractive index, dynamic light scattering or UV-VIS detector can be used. Alternatively, in order to track the activity in each fraction, a specific enzymatic activity or the like can be used.

As the method for purifying the microparticles, the method described in [0034] to [0064] of JP2019-524824A may be used.

### <Other Components>

The agent for use in improving cancer cachexia of the invention may contain another component in addition to the microparticles depending on the species of the animal as the subject of the administration or the purpose as long as the effects of the invention are not impaired. Examples of the other component include nutritional components, antibiotics, cytokines, protectants, carriers, excipients, disintegrating agents, buffers, emulsifiers, suspending agents, soothing agents, stabilizers, preservatives, antiseptics and the like.

Examples of the nutritional components include fatty acids, vitamins and the like.

Examples of the antibiotics include penicillin, streptomycin, gentamicin and the like.

The carriers may be materials known as pharmaceutically acceptable carriers.

The agent for use in improving cancer cachexia of the invention may be the culture supernatant itself of dental pulp-derived stem cells or the microparticles themselves or may be a pharmaceutical composition further containing a pharmaceutically acceptable carrier or excipient or the like. The purpose of the pharmaceutical composition is to promote administration of the culture supernatant of dental pulp-derived stem cells or the microparticles to the subject of the administration.

The pharmaceutically acceptable carrier is preferably a carrier (including a diluent) which does not cause significant irritation in the subject of the administration and which does not inhibit the biological activity and the characteristics of the administered composition. Examples of the carrier include propylene glycol, (physiological) saline, an emulsion, a buffer, a medium such as DMEM and RPMI and a low-temperature preservation medium containing a component for removing free radicals.

The agent for use in improving cancer cachexia of the invention may contain the active ingredient of a conventionally known agent for improving cancer cachexia. One skilled in the art can appropriately change depending on the use, the subject of the administration and the like.

The agent for use in improving cancer cachexia of the invention preferably does not contain specific substances.

For example, the agent for use in improving cancer cachexia of the invention preferably does not contain dental pulp-derived stem cells.

Moreover, the agent for use in improving cancer cachexia of the invention preferably does not contain MCP-1. In this regard, however, cytokines other than MCP-1 may be contained. Examples of the other cytokines include those described in [0014] to [0020] of JP2018-023343A and the like.

The agent for use in improving cancer cachexia of the invention preferably does not contain Siglec-9. In this regard, however, sialic acid-binding immunoglobulin-like lectins other than Siglec-9 may be contained.

The agent for use in improving cancer cachexia of the invention preferably does not substantially contain serum (fetal bovine serum, human serum, sheep serum or the like). Moreover, the agent for use in improving cancer cachexia of the invention preferably does not substantially contain the conventional serum replacements such as knockout serum replacement (KSR).

The amounts (solid amounts) of the other components in the agent for use in improving cancer cachexia of the invention are each preferably 1 mass% or less, more preferably 0.1 mass% or less, particularly preferably 0.01 mass% or less.

### <Production Method of Agent for Use in Improving Cancer Cachexia>

The method for producing the agent for use in improving cancer cachexia of the invention is not particularly restricted.

The agent for use in improving cancer cachexia may be prepared by preparing a culture supernatant of dental pulp-derived stem cells by the above method. The agent for use in improving cancer cachexia of the invention may be prepared by subsequently purifying microparticles from the culture supernatant of dental pulp-derived stem cells. Alternatively, the agent for use in improving cancer cachexia of the invention may be prepared by purifying microparticles from a commercially purchased culture supernatant of dental pulp-derived stem cells. The agent for use in improving cancer cachexia of the invention may also be prepared by receiving a composition containing a culture supernatant of dental pulp-derived stem cells which has been disposed of (or by appropriately purifying such a composition) and purifying microparticles from the composition.

The final form of the agent for use in improving cancer cachexia of the invention is not particularly restricted. For example, the agent for use in improving cancer cachexia of the invention may be in a form in which the culture supernatant of dental pulp-derived stem cells is packed in a container, a form in which the microparticles are packed in a container with a solvent or a dispersion medium, a form in which the microparticles are formed into gel with gel and packed in a container, a form in which the culture supernatant of dental pulp-derived stem cells or the microparticles are solidified by freezing and/or drying and formulated or packed in a container or another form. Examples of the container include a tube, a centrifuge tube, a bag or the like which is suitable for cryopreservation. The freezing temperature can be, for example, -20°C to - 196°C.

### [Method for Improving Cancer Cachexia]

The method for improving cancer cachexia includes administering an effective amount of the agent for use in improving cancer cachexia of the invention to a subject with cancer cachexia.

The step of administering the agent for use in improving cancer cachexia of the invention to a subject with cancer cachexia is not particularly restricted.

The administration method can be spraying or inhalation to the oral cavity, the nasal cavity or the respiratory tract, infusion, local administration, nasal drops or the like and is preferably little invasive. The local administration method is preferably an injection. Another preferable method is electroporation, in which voltage (electric pulses) is applied to the skin surface to create temporal fine holes in the cell membrane, allowing the active ingredient to penetrate to the dermis layer, which cannot be reached by normal care. The local administration may be intravenous administration, intraarterial administration, intraportal administration, intradermal administration, subcutaneous administration, intramuscular administration, intraperitoneal administration or the like and is more preferably intraarterial administration, intravenous administration, subcutaneous administration or intraperitoneal administration.

The agent for use in improving cancer cachexia of the invention which has been administered to a subject with cancer cachexia circulates in the body of the subject and may reach a certain tissue.

The number of administrations and the intervals of administration are not particularly restricted. The number of administrations can be once a week or more and is preferably five times or more, more preferably six times or more, particularly preferably seven times or more. The intervals of administration are preferably an hour to a week, more preferably half a day to a week, particularly preferably a day (once a day). However, the number and the intervals can be appropriately adjusted in accordance with the species of the subject of the administration and the symptom of the subject of the administration.

The agent for use in improving cancer cachexia of the invention is preferably administered to a subject with cancer cachexia once a week or more for a therapeutically effective period. When the subject of the administration is a human, the number of administrations per week is preferably higher, and the agent is preferably administered five times a week or more for a therapeutically effective period or preferably administered every day.

When a culture supernatant of dental pulp-derived stem cells having a concentration of 2.0×10⁹ cells/mL is used, the dose in a mouse model is preferably 0.1 to 5 mL per mouse (about 25 g), more preferably 0.3 to 3 mL, further particularly preferably 0.5 to 1 mL.

When microparticles having a concentration of 0.1×10⁸ particles/µg are used, the dose in a mouse model is preferably 1 to 50 µg per mouse (about 25 g), more preferably 3 to 30 µg, further particularly preferably 5 to 25 µg.

A preferable range of the dose per body weight for another animal can be calculated using the proportional relation from the dose per body weight (about 25 g) of the model mouse. However, the dose can be appropriately adjusted in accordance with the symptom of the subject of the administration.

The animal (species) as the subject to which the agent for use in improving cancer cachexia of the invention is administered is not particularly restricted. The animal as the subject to which the agent for use in improving cancer cachexia of the invention is administered is preferably a mammal, a bird (chicken, quail, duck or the like) or fish (salmon, trout, tuna, bonito or the like). The mammal may be a human or a nonhuman mammal but is particularly preferably a human. The nonhuman mammal is more preferably a cow, a pig, a horse, a goat, a sheep, a monkey, a dog, a cat, a mouse, a rat, a guinea pig or a hamster.

The agent for use in improving cancer cachexia of the invention may be used in combination with a conventionally known agent for improving cancer cachexia. Examples

The characteristics of the invention are explained further specifically referring to Examples and Comparative Examples or a Reference Example below. The materials, the amounts, the proportions, the contents of treatments, the procedures of treatments and the like shown in the Examples below can be appropriately changed as long as such changes do not go beyond the intention of the invention.

### [Example 1]

### <Experiment Protocol>

In accordance with the experiment protocol shown in FIG. 1, after administering samples to cancer cachexia model mice, whole blood samples were taken, and improvement of cachexia was evaluated.

### <Preparation of Lethal Cancer Cachexia Model Mice>

To seven-week-old nude mice (Balb/c-nu/nu) used as normal mice, 1×10⁶ cells of B16-F10-luc-G5 cells (B16-luc cells) were subcutaneously transplanted, and lethal cancer cachexia model mice were prepared. The conditions are severe conditions under which all the mice die within 18 days.

### <Preparation of Culture Supernatant of Dental Pulp-Derived Stem Cells>

A culture supernatant of human deciduous dental pulp stem cells was prepared according to the method described in Example 6 of JP6296622B except that DMEM medium was used instead of the DMEM/HamF12 mixture medium, and the culture supernatant was collected. For the primary culture, fetal bovine serum (FBS) was added to the culture. In the passaging culture, the supernatant was taken from the passaged culture solution which was cultured using the primary culture solution in such a manner that FBS would not be contained, and a culture supernatant of deciduous dental pulp stem cells was thus prepared. Here, DMEM is Dulbecco's modified Eagle's medium, and F12 is Ham's F12 medium.

The prepared culture supernatant of dental pulp-derived stem cells was used as the sample agent for use in improving cancer cachexia of Example 1.

### <Sample Administration>

The sample of Example 1 at 0.5 mL (1.0×10⁹ particles in terms of exosomes) per mouse was administered to the cancer cachexia model mice from the tail vein on the treatment day (day 0) on which the cancer cachexia model mice were prepared and seven days and 14 days after the treatment day.

### [Example 2]

### <Preparation of Exosomes>

Exosomes of dental pulp-derived stem cells were purified from the culture supernatant of dental pulp-derived stem cells obtained in Example 1 by the following method.

After filtering the culture supernatant of deciduous dental pulp stem cells (100 mL) with a filter having a pore size of 0.22 micrometers, the solution was centrifuged for 60 minutes at 4°C at 100000× g. The supernatant was decanted, and the exosome-concentrated pellets were re-suspended in phosphate-buffered saline (PBS). The re-suspended sample was centrifuged for 60 minutes at 100000× g. The pellets were again recovered from the bottom of the centrifuge tube as the concentrated sample (about 100 µL). The protein concentration was determined using a micro BSA protein assay kit (Pierce, Rockford, IL). The composition containing exosomes (concentrated solution) was stored at -80°C.

The composition containing the exosomes purified from the culture supernatant of dental pulp-derived stem cells was used as the sample agent for improving cancer cachexia of Example 2.

The average particle sizes and the concentrations of the microparticles contained in the agents for use in improving cancer cachexia of the Examples were evaluated.

The average particle sizes of the microparticles contained in the agents for use in improving cancer cachexia of the Examples were 50 to 150 nm.

The agents for use in improving cancer cachexia of the Examples were exosome solutions having a high concentration of 1.0×10⁹ particles/mL or more, and in particular, the agent for use in improving cancer cachexia of Example 1 was an exosome solution having a high concentration of 2.0×10⁹ particles/mL.

Moreover, the components of the obtained agents for use in improving cancer cachexia of the Examples were analyzed by a known method. As a result, it was found that the agents for use in improving cancer cachexia of the Examples did not contain any stem cells of dental pulp-derived stem cells, did not contain MCP-1 and did not contain Siglec-9. It was thus found that an active ingredient which is different from MCP-1 and Siglec-9, which are active ingredients of a culture supernatant of mesenchymal stem cells, and from analogs thereof is the active ingredient of the agents for use in improving cancer cachexia of the Examples.

In the same manner as in Example 1 except for administering 20 µg (2.0×10⁸ particles in terms of exosomes) of the sample of Example 2, instead of the sample of Example 1, to the cancer cachexia model mice prepared in Example 1, the sample was administered.

### [Comparative Example 1]

In the same manner as in Example 1 except for administering the same amount, namely 0.5 mL, of physiological saline as the sample of Comparative Example 1, instead of the sample of Example 1, to the cancer cachexia model mice prepared in Example 1, the sample was administered.

### [Evaluation]

### (Evaluation of Weight Loss)

The body weights of the cancer cachexia model mice were measured on the treatment day (day 0) on which the cancer cachexia model mice were prepared and seven days, 14 days and 21 days after the treatment day. Each group was with n=10, and in Example 1 for example, the average body weight of the ten mice to which the sample of Example 1 was administered were determined.

Seven-week-old nude mice (Balb/c-nu/nu) were used for a Reference Example with normal mice. Using the body weights of the normal mice of Reference Example 1 as the control (100%), the body weights (% based on the control) of the 10 mice to which the sample of any of the Examples and the Comparative Example was administered were determined. The obtained results are shown in FIG. 2.

From the results in FIG. 2, it was found that the weight loss can be suppressed by administering the agents for use in improving cancer cachexia of the invention to cancer cachexia model mice compared to the case with administration of physiological saline.

From the results, it is expected that the agent for use in improving cancer cachexia of the invention suppresses weight loss of a patient with cachexia and thus can maintain also the skeletal muscle mass or the like, improve anemia and improve the QOL and the ADL.

In this regard, the mechanism of such action of suppressing weight loss is believed to be through suppression of inflammatory cytokines, suppression of a cytokine storm and the like from the results of the Examples described below.

### (Evaluation of Hemocytes and the Like in Blood)

Whole blood samples were taken 21 days after the treatment day (day 0) on which the cancer cachexia model mice were prepared. Then, the red blood cell counts (unit: 10000/µL), the hemoglobin levels (blood pigments, unit g/dL), the platelet counts (unit: 10000/µL) and the hematocrits (%) in the blood were measured. The obtained results are shown in Table 1.

From the results in Table 1, it was found that the red blood cell count, the hemoglobin level (blood pigments), the platelet count and the hematocrit can be maintained high by administering the agents for use in improving cancer cachexia of the invention to cancer cachexia model mice compared to the case with administration of physiological saline.

Moreover, an anemia-like symptom was observed in the cancer cachexia model mice of Comparative Example 1, to which physiological saline was administered, while no particular anemia-like symptom was observed in the cancer cachexia model mice to which the agents for use in improving cancer cachexia of the invention were administered.

From the results, it is expected that the agent for use in improving cancer cachexia of the invention improves anemia of a patient with cachexia and thus can improve the QOL and the ADL.

**[Table 1]**

| | Comparative Example 1 Physiological Saline | Example 1 Culture Supernatant | Example 2 Exosomes |
|---|---|---|---|
| Red Blood Cell Count | 572.5 ± 23.2 | 912.9 ± 18.5 | 898.1 ± 55.3 |
| Hemoglobin Level (Blood Pigments) | 8.9 ± 0.3 | 15.2 ± 3.4 | 10.9 ± 4.0 |
| Platelet Count | 22.0 ± 5.3 | 50.1 ± 6.7 | 60.2 ± 4.4 |
| Hematocrit | 33.2 ± 0.9 | 44.7 ± 3.6 | 50.1 ± 2.7 |

### (Evaluation of Blood Cytokine Amounts)

Whole blood samples were taken 21 days after the treatment day (day 0) on which the cancer cachexia model mice were prepared. Then, the amounts of IL-6 and TNF-α in the blood were measured with Immune Monitoring 48-Plex Mouse ProcartaPlex (registered trademark) Panel (manufactured by Invitrogen). In addition, whole blood samples were taken also from the normal mice used in Reference Example 1, and the amounts of the cytokines contained in the blood were measured in the same manner. The obtained results are shown in FIG. 3 and FIG. 4.

From the results in FIG. 3 and FIG. 4, it was found that increases in the IL-6 and TNF-α concentrations in the blood can be suppressed by administering the agents for use in improving cancer cachexia of the invention to cancer cachexia model mice compared to the case with administration of physiological saline.

From the results, it is expected that the agent for use in improving cancer cachexia of the invention can improve the QOL and the ADL, for example, by increasing appetite through suppression of the production of inflammatory cytokines in a patient with cachexia or by improving metabolic abnormality through suppression of inflammatory reaction at the tumor site or suppression of a cytokine storm.

### (Evaluation (1) of Survival Rates in Lethal Cancer Cachexia Mouse Model)

The survival rates of the mice of the groups (n=10) were observed every other day from the treatment day (day 0) on which the cancer cachexia model mice were prepared. The obtained results are shown in FIG. 5.

From the results in FIG. 5, it was found that, when the agents for use in improving cancer cachexia of the invention with the exosome concentrations of Examples 1 and 2 are administered to cancer cachexia model mice, the average survival period (days) can be slightly extended, and the survival rate can be slightly increased, compared to the case with administration of physiological saline.

### [Example 11]

### (Evaluation (2) of Survival Rates in Lethal Cancer Cachexia Mouse Model)

The survival rate of Example 11 was evaluated in the same manner as in Example 2 except that 25 µg (2.5×10⁸ particles in terms of exosomes) of the sample of Example 2 (exosomes purified from a culture supernatant of dental pulp-derived stem cells) was administered to cancer cachexia model mice. Here, the dose of the sample of Example 11 is 1.25 times the dose of the sample of Example 2. Example 11 was conducted with n=10. The obtained results are shown in FIG. 6.

From the results in FIG. 6, it was found that, when the agent for use in improving cancer cachexia of the invention with an exosome concentration increased to that of Example 11 is administered to cancer cachexia model mice, the average survival period (days) can be extended, and the survival rate can be increased compared to the case with administration of physiological saline.

### [Example 21]

### <In Vitro Observation of Cytokine Storm Suppression>

For the purpose of supplementing the results of evaluation of the blood cytokine amounts of Example 2, a cell model in which a cytokine storm was induced was used instead of the cancer cachexia model mice, and the effect of suppressing a cytokine storm of the agent for use in improving cancer cachexia of the invention was observed.

A cytokine storm was induced using human peripheral-blood mononuclear cells (PBMCs), and the cytokine storm suppression effect of the sample of Example 2 was observed *in vitro* by the following method.

Human PBMCs were cultured and stimulated with a cytokine storm inducer, Concanavalin A (Con-A, manufactured by FUJIFILM Wako Pure Chemical Corporation), and thus a cytokine storm was induced.

The system was subjected to treatment of adding one kind of the sample of Example 2 and physiological saline as a control, and the amounts of cytokines contained in the culture supernatants of human PBMCs were measured with a cytokine measurement ELISA kit (manufactured by R&D Systems, product name Quantikine ELISA kit). The quantified cytokines are IL-2, IL-4, IL-6, IL-10, IL-17, IFNγ and TNFα.

The addition of the sample of Example 2 was treated to achieve 100 particles per PBMC. The cytokine amounts were measured 48 hours after the treatment with the sample of Example 2 (N=6).

The obtained results are shown in Table 2 below.

**[Table 2]**

| | Control | Example 21 |
|---|---|---|
| Microparticles | None (Physiological Saline) | Dental Pulp-Derived Stem Cells Exosomes |
| IL-2 (pg/mL) | 28.9 ± 6.3 | 11.5 ± 3.1 |
| IL-4 (pg/mL) | 19.9 ± 12.0 | 5.7 ± 4.1 |
| IL-6 (pg/mL) | 112.0 ± 23.1 | 10.6 ± 5.5 |
| IL-10 (pg/mL) | 125.2 ± 33.9 | 100.1 ± 12.5 |
| IL-17 (pg/mL) | 141.3 ± 25.7 | 11.3 ± 2.0 |
| IFNγ (pg/mL) | 3045.5 ± 12.2 | 92.9 ± 4.3 |
| TNFα (pg/mL) | 1022 ± 29.4 | 151.8 ± 6.6 |

| | | |
|---|---|---|
| (N=6) | | |

### [Example 22]

### <In Vivo Observation of Cytokine Storm Suppression>

A cytokine storm was induced in a mouse model, and the cytokine storm suppression effect of the sample of Example 2 was observed *in vivo* by the following method.

Six eight-week-old C57BL/6J mice were used per group.

Lipopolysaccharide (LPS), which is a cytokine storm inducer, was intraperitoneally administered to the mice at 20 mg/kg body weight. Here, administration of lipopolysaccharide (LPS, endotoxin) can cause symptoms of sepsis such as an increased inflammatory cytokine concentration and induce a cytokine storm (Medical Hypotheses (2020) 144, 109865).

Immediately after the lipopolysaccharide administration, one kind of the sample of Example 2 (purified by ultracentrifugation) and physiological saline as a control, in the same amount of 10 µg, was administered to the mice from the tail vein. The dose of the sample of Example 21 is 0.5 times the dose of the sample of Example 2.

Whole blood samples were taken after 24 hours, and the serum of the mice with the cytokine storm was fractionated. The amounts of cytokines contained in the serum of the mice with the cytokine storm were measured with Immune Monitoring 48-Plex Mouse ProcartaPlex (registered trademark) Panel (manufactured by Invitrogen). In addition, whole blood samples were taken also from the normal mice before the lipopolysaccharide administration, and the amounts of cytokines contained in the serum were measured in the same manner. The quantified cytokines are IL-2, IL-4, IL-6, IL-10, IL-17, IFNγ and TNFα.

The obtained results are shown in Table 3 below.

**[Table 3]**

| | Normal Mouse | Control | Example 22 |
|---|---|---|---|
| Microparticles | None (Cytokine Storm Uninduced) | None (Physiological Saline) | Dental Pulp-Derived Stem Cells Exosomes |
| IL-2 (pg/mL) | ND | 3420 ± 44 | 158 ± 21 |
| IL-4 (pg/mL) | ND | 2005 ± 27 | 509 ± 20 |
| IL-6 (pg/mL) | 31 ± 2 | 1502 ± 46 | 607 ± 18 |
| IL-10 (pg/mL) | 19 ± 9 | 4438 ± 97 | 1221 ± 45 |
| IL-17 (pg/mL) | ND | 2543 ± 47 | 1022 ± 36 |
| IFNγ (pg/mL) | ND | 986 ± 21 | 40 ± 3 |
| TNFα (pg/mL) | 10 ± 4 | 202 ± 16 | 14 ± 2 |

| | | | |
|---|---|---|---|
| ND: not detected (N=6) | | | |

From Table 2 and Table 3 above, it was found that the sample of Example 2, which is an agent for use in improving cancer cachexia of the invention, can also significantly suppress the amounts of cytokines other than IL-6 and TNFα in the blood and/or the serum and can suppress a cytokine storm when the sample is administered to an animal (mouse) with higher cytokine amounts due to an induced cytokine storm, compared to the control with administration of physiological saline. From the results, it is expected that the agent for use in improving cancer cachexia of the invention can improve weight loss, anemia and undernutrition and improve the QOL and the ADL, for example, by increasing appetite and improving metabolic abnormality through suppression of inflammatory cytokines and a cytokine storm of a patient with cachexia.

### [Comparative Examples A to D]

### <Comparison Between Culture Supernatant and Exosomes of Dental Pulp-Derived Stem Cells of Examples 1 and 2 and Culture Supernatant or Exosomes of Umbilical Cord-Derived Stem Cells>

It was examined whether the production of IL-6 from active macrophages, which is a main cause of a cytokine storm, would be suppressed by causing activation of mouse-derived macrophage cells RAW264.7 by stimulation with LPS and then adding a culture supernatant or exosomes of stem cells.

DMEM and 10% FBS (Cat.No.: DSDSM101) were used for a culture solution. RAW264.7 (1.2×10⁶/mL, TIB-71, purchased from ATCC) was prepared in a 96-well plate and cultured in the culture solution, and when the cells became 90% subconfluent, LPS (100 ng/mL, ALX-581-007-L002, manufactured by Cosmo Bio Co., Ltd.) and the sample agents for use in improving cancer cachexia of the Examples and the Comparative Examples were added. The cells were cultured for 18 hours at 37°C in 5% CO₂. After the culture, the amounts of IL-6 released into the culture solutions were measured with a mouse IL-6 measurement ELISA kit (KE10007, manufactured by Cosmo Bio Co., Ltd.). The obtained measured values (the averages of N=2) are shown in FIG. 7.

Moreover, as controls, a same test was conducted using untreated cells without administration of LPS (Comparative Example A) and cells to which only LPS was administered (Comparative Example B) without adding the sample agents for use in improving cancer cachexia, and the results thereof are also shown in FIG. 7.

As the sample agents for use in improving cancer cachexia, the agent for use in improving cancer cachexia used in Example 1 of the specification of the present application (a culture supernatant of dental pulp-derived stem cells), the agent for use in improving cancer cachexia used in Example 2 of the specification of the present application (exosomes of dental pulp-derived stem cells), a culture supernatant of umbilical cord-derived stem cells of Comparative Example C and exosomes of Comparative Example D (exosomes of umbilical cord-derived stem cells), which were prepared according to [0053] from a culture supernatant of umbilical cord-derived stem cells, were used. The exosomes were separated by ultracentrifugation, and 1000 exosomes were used per mouse-derived macrophage cell.

From FIG. 7, the agent for use in improving cancer cachexia of Example 1 using a culture supernatant of dental pulp-derived stem cells exhibited a significantly advantageous effect in cytokine suppression in *in vitro* cells with an induced cytokine storm, compared to the case using the culture supernatant of umbilical cord-derived stem cells (Comparative Example C). Moreover, the agent for use in improving cancer cachexia of Example 2 using microparticles (exosomes) obtained from a culture supernatant of dental pulp-derived stem cells exhibited a significantly advantageous effect in cytokine suppression in *in vitro* cells with an induced cytokine storm, compared to the case using microparticles (exosomes) obtained from a culture supernatant of umbilical cord-derived stem cells (Comparative Example D).

Here, in *in vitro* Example 21, the effect of suppressing a cytokine storm of the agent for use in improving cancer cachexia of the invention was examined *in vitro* using animal cells with an induced cytokine storm instead of the cancer cachexia model mice, for the purpose of supplementing the evaluation results of suppression of the blood cytokine amounts (IL-6 and the like) in the *in vivo* cancer cachexia model mice of Example 2. That is, it was found that, by evaluating the suppression of cytokines such as IL-6 in *in vitro* cells with an induced cytokine storm in this manner, the effect of improving cancer cachexia in an *in vivo* animal such as cancer cachexia model mice (improving weight loss, anemia and undernutrition and improving the QOL and the ADL by suppressing inflammatory cytokines or a cytokine storm and thus increasing appetite or improving metabolic abnormality, for example) can be evaluated.

Accordingly, from the results of the comparison test between Example 1 or 2 and Comparative Examples A to D (the evaluation results of suppression of cytokines such as IL-6 in *in vitro* cells with an induced cytokine storm), it was found that a culture supernatant of dental pulp-derived stem cells and exosomes obtained therefrom have an effect of significantly improving cancer cachexia compared to a culture supernatant of umbilical cord-derived stem cells and exosomes obtained therefrom.

## Claims

1. An agent for use in improving cancer cachexia by administration to a subject with cancer cachexia
which is a composition containing a culture supernatant of dental pulp-derived stem cells,
wherein the culture supernatant contains microparticles derived from the dental pulp-derived stem cells,
wherein the microparticle is at least one kind selected from the group consisting of exosomes, membrane vesicles, ectosomes, exovesicles and microvesicles, more preferably exosomes.

2. An agent for use in improving cancer cachexia by administration to a subject with cancer cachexia
which is a composition containing microparticles derived from a culture supernatant of dental pulp-derived stem cells,
wherein the microparticle is at least one kind selected from the group consisting of exosomes, membrane vesicles, ectosomes, exovesicles and microvesicles, more preferably exosomes,
wherein the composition does not contain the culture supernatant from which the microparticles have been removed.

3. The agent for use in improving cancer cachexia according to claim 1 or 2, wherein the microparticles are exosomes.

4. The agent for use in improving cancer cachexia according to any one of claims 1 to 3, wherein the improvement of cancer cachexia is treating weight loss of the subject with cancer cachexia.

5. The agent for use in improving cancer cachexia according to claim 4, wherein the weight loss of the subject with cancer cachexia is 2% or more in the past 14 days.

6. The agent for use in improving cancer cachexia according to any one of claims 1 to 5, wherein the improvement of cancer cachexia is treating anemia of the subject with cancer cachexia.

7. The agent for use in improving cancer cachexia according to claim 6, wherein the improvement of cancer cachexia is suppressing decreases in red blood cell count, hemoglobin level and hematocrit of the subject with cancer cachexia.

8. The agent for use in improving cancer cachexia according to any one of claims 1 to 7, wherein the improvement of cancer cachexia is suppressing increases in IL-6 concentration and TFN-α concentration in blood of the subject with cancer cachexia.

9. The agent for use in improving cancer cachexia according to any one of claims 1 to 8 wherein the agent is administered to the subject with cancer cachexia once a week or more for a therapeutically effective period.

10. The agent for use in improving cancer cachexia according to any one of claims 1 to 9, wherein the subject with cancer cachexia is a nonhuman animal.

## Patentansprüche

1. Ein Mittel zur Verwendung bei der Verbesserung der Krebskachexie durch Verabreichung an einen Patienten mit Krebskachexie,
wobei es sich um eine Zusammensetzung handelt, die einen Kulturüberstand von aus Zahnmark gewonnenen Stammzellen enthält,
wobei der Kulturüberstand Mikropartikel enthält, die aus den aus Zahnmark gewonnenen Stammzellen stammen,
wobei das Mikropartikel mindestens ein Typ ist ausgewählt aus der Gruppe bestehend aus Exosomen, Membranvesikeln, Ektosomen, Exovesikeln und Mikrovesikeln, vorzugsweise Exosomen.

2. Ein Mittel zur Verwendung bei der Verbesserung von Krebskachexie durch Verabreichung an einen Patienten mit Krebskachexie,
wobei es sich um eine Zusammensetzung handelt, die Mikropartikel enthält, die aus einem Kulturüberstand von aus Zahnmark gewonnenen Stammzellen stammen,
wobei das Mikropartikel mindestens ein Typ ist ausgewählt aus der Gruppe bestehend aus Exosomen, Membranvesikeln, Ektosomen, Exovesikeln und Mikrovesikeln besteht, vorzugsweise Exosomen,
wobei die Zusammensetzung keinen Kulturüberstand enthält, aus dem die Mikropartikel entfernt wurden.

3. Das Mittel zur Verwendung bei der Verbesserung der Krebskachexie gemäß Anspruch 1 oder 2, wobei die Mikropartikel Exosomen sind.

4. Das Mittel zur Verwendung bei der Verbesserung der Krebskachexie gemäß einem der Ansprüche 1 bis 3, wobei die Verbesserung der Krebskachexie die Behandlung des Gewichtsverlusts des Patienten mit Krebskachexie umfasst.

5. Das Mittel zur Verwendung bei der Verbesserung von Krebskachexie gemäß Anspruch 4, wobei der Gewichtsverlust des Patienten mit Krebskachexie in den letzten 14 Tagen 2 % oder mehr beträgt.

6. Das Mittel zur Verwendung bei der Verbesserung der Krebskachexie gemäß einem der Ansprüche 1 bis 5, wobei die Verbesserung der Krebskachexie die Behandlung der Anämie des Patienten mit Krebskachexie umfasst.

7. Das Mittel zur Verwendung bei der Verbesserung der Krebskachexie gemäß Anspruch 6, wobei die Verbesserung der Krebskachexie die Unterdrückung der Abnahme der Anzahl roter Blutkörperchen, des Hämoglobinspiegels und des Hämatokritwerts des Patienten mit Krebskachexie umfasst.

8. Das Mittel zur Verwendung bei der Verbesserung der Krebskachexie gemäß einem der Ansprüche 1 bis 7, wobei die Verbesserung der Krebskachexie darin besteht, den Anstieg der IL-6-Konzentration und der TFN-α-Konzentration im Blut des Patienten mit Krebskachexie zu unterdrücken.

9. Das Mittel zur Verwendung bei der Verbesserung von Krebskachexie gemäß einem der Ansprüche 1 bis 8, wobei das Mittel dem Patienten mit Krebskachexie einmal pro Woche oder öfter über einen therapeutisch wirksamen Zeitraum verabreicht wird.

10. Das Mittel zur Verwendung bei der Verbesserung von Krebskachexie gemäß einem der Ansprüche 1 bis 9, wobei der Patient mit Krebskachexie ein nichtmenschliches Tier ist.

## Revendications

1. Agent destiné à être utilisé pour améliorer la cachexie cancéreuse par administration à un sujet atteint de cachexie cancéreuse
qui est une composition contenant un surnageant de culture de cellules souches dérivées de la pulpe dentaire,
dans lequel le surnageant de culture contient des microparticules dérivées des cellules souches dérivées de la pulpe dentaire,
dans lequel la microparticule est au moins un type choisi dans le groupe constitué des exosomes, les vésicules membranaires, les ectosomes, les exovésicules et les microvésicules, de préférence les exosomes.

2. Agent destiné à être utilisé pour améliorer la cachexie cancéreuse par administration à un sujet atteint de cachexie cancéreuse
qui est une composition contenant des microparticules dérivées d'un surnageant de culture de cellules souches dérivées de la pulpe dentaire,
dans lequel la microparticule est au moins un type choisi dans le groupe constitué des exosomes, les vésicules membranaires, les ectosomes, les exovésicules et les microvésicules, de préférence les exosomes,
dans lequel la composition ne contient pas le surnageant de culture dont les microparticules ont été retirées.

3. Agent destiné à être utilisé pour améliorer la cachexie cancéreuse selon la revendication 1 ou 2, dans lequel les microparticules sont des exosomes.

4. Agent destiné à être utilisé pour améliorer la cachexie cancéreuse selon l'une quelconque des revendications 1 à 3, dans lequel l'amélioration de la cachexie cancéreuse consiste à traiter la perte de poids du sujet atteint de cachexie cancéreuse.

5. Agent destiné à être utilisé pour améliorer la cachexie cancéreuse selon la revendication 4, dans lequel la perte de poids du sujet atteint de cachexie cancéreuse est de 2 % ou plus au cours des 14 derniers jours.

6. Agent destiné à être utilisé pour améliorer la cachexie cancéreuse selon l'une quelconque des revendications 1 à 5, dans lequel l'amélioration de la cachexie cancéreuse consiste à traiter l'anémie du sujet atteint de cachexie cancéreuse.

7. Agent destiné à être utilisé pour améliorer la cachexie cancéreuse selon la revendication 6, dans lequel l'amélioration de la cachexie cancéreuse consiste à supprimer les diminutions du nombre de globules rouges, du taux d'hémoglobine et de l'hématocrite du sujet atteint de cachexie cancéreuse.

8. Agent destiné à être utilisé pour améliorer la cachexie cancéreuse selon l'une quelconque des revendications 1 à 7, dans lequel l'amélioration de la cachexie cancéreuse consiste à supprimer les augmentations de la concentration en IL-6 et de la concentration en TFN-α dans le sang du sujet atteint de cachexie cancéreuse.

9. Agent destiné à être utilisé pour améliorer la cachexie cancéreuse selon l'une quelconque des revendications 1 à 8, dans lequel l'agent est administré au sujet atteint de cachexie cancéreuse une fois par semaine ou plus pendant une période thérapeutiquement efficace.

10. Agent destiné à être utilisé pour améliorer la cachexie cancéreuse selon l'une quelconque des revendications 1 à 9, dans lequel le sujet atteint de cachexie cancéreuse est un animal non humain.
